# EUROPEAN PATENT SPECIFICATION

(11) **EP 2 672 904 B1**
(45) Date of publication and mention of the grant of the patent: **31.05.2017**
(21) Application number: 12708371.5
(22) Date of filing: 10.02.2012
(51) Int. Cl.: A61B 17/3211, B26B 3/06, B26B 29/02, A61B 90/00

(54) **IMPROVEMENTS IN HAND-HELD CUTTING IMPLEMENTS**
VERBESSERUNGEN BEI TRAGBAREN SCHNEIDVORRICHTUNGEN
PERFECTIONNEMENTS APPORTÉS À DES ACCESSOIRES COUPANTS À MAIN

(30) Priority: 10.02.2011 GB 201102323
(43) Date of publication of application: 18.12.2013
(73) Proprietor: George, Samuel, Weybridge, Surrey KT13 9SB (GB); Musleh, Wael, Surrey KT13 9SB (GB)
(72) Inventor: George, Samuel, Weybridge, Surrey KT13 9SB (GB); Musleh, Wael, Surrey KT13 9SB (GB)
(74) Representative: Cummings, Sean Patrick
(86) International application number: PCT/GB2012/050308
(87) International publication number: WO 2012/107781

(56) References cited:
- WO-A1-2004/002335
- US-A- 5 417 704
- US-A1- 2006 212 058

## Description

The present invention relates to hand-held cutting instruments such as scalpels, and in particular to resheathable safety instruments.

It is well known that scalpels present a hazard to medical staff during use. In particular, passing scalpels from one person to another, especially in high pressured, fast-paced environments such as an operating theatre, causes a significant number of injuries. In addition to the pain and bleeding that will inevitably result, the longer-term effects of these injuries can be potentially devastating. The injury site is at risk of infection, and if the scalpel has been used prior to injury there is a risk of transferring fatal bacteria, or viral infections such as HIV or Hepatitis. It is therefore desirable to provide a scalpel in which the blade may be covered when the instrument is not in use, in order to prevent such injuries.

There have been a number of attempts to provide such safety scalpels. Fixed-blade safety scalpels comprise a sheath that can be moved to cover or expose the blade as required. In the simplest form the sheath is a cover that can be removed from the scalpel during use, and replaced after use. However, covers such as these are awkward to remove and replace accurately, and are easily misplaced. The result is that such covers are often abandoned during use of the scalpel, negating their safety effects. Even if such covers are used, removal and replacement of the cover presents a significant hazard in itself due to the risk of sharps injury.

In more sophisticated arrangements, a retractable sheath may be moved to cover or expose the blade as required. Often, the sheath is biased to cover the blade and may be moved to expose the blade upon pressing a release button. WO2008/132762 and US2002/0143352 are examples of such retractable-sheath scalpels. An alternative design is a retractable-blade scalpel in which the blade is retractable into the handle of the instrument to shield it as required. Similarly, the blade may be biased into the retracted position and may be extended upon pressing a release button.

Although retractable-sheath and retractable-blade scalpels do not have the disadvantages associated with a removable cover, they present other problems. Pressing a release button or buttons on the scalpel may be cumbersome for the user and so discourages effective use of the safety features. Accidental depression of a release button can also occur, and can lead to inadvertent re-sheathing or retraction of the blade during use. This can present a significant hazard; indeed it may result in injuries of the sort that the safety features are intended to prevent. Furthermore, stability and reliability in use of surgical instruments is of paramount importance, and unexpected sheathing or retraction of the blade can lead to errors in surgical procedures.

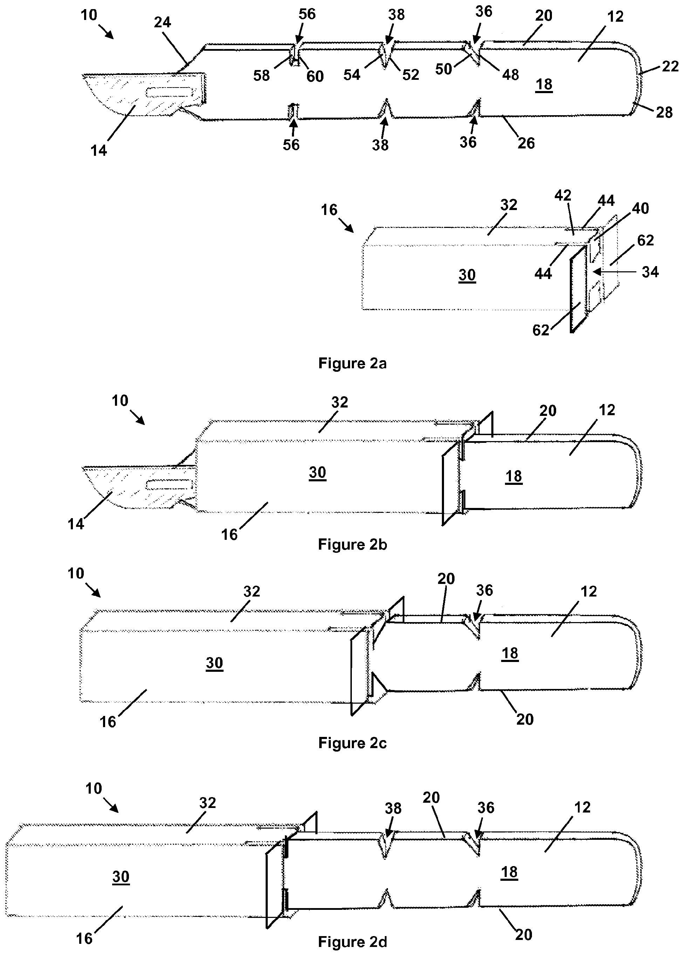

Perhaps the most significant disadvantages of retractable-sheath and retractable-blade scalpels result from their complex and bulky mechanisms, which for example involve biasing springs, locking mechanisms and release buttons. This means that such instruments are complicated and costly to produce. Cost-effectiveness is particularly important because the majority of scalpels are treated as disposable instruments. US5417704, WO2004/002335 and US2006/0212058 are examples of such complex scalpels. For example, the scalpel disclosed in US5417704 includes a handle with grooves, a keyway and ribs on either side portion (see Figure 2) and a cover with keys and latching detents to engage with the handle (see Figure 3). The scalpel disclosed in US2006/0212058 comprises an arrangement whereby a plurality of notches on the lower edge of a handle and a blade carrier for engagement with a retention tooth on a blade cover define at least protective and use positions of the scalpel, the retention tooth being disengaged from said notches to allow movement between protective and use positions only by depression of a release button (see Figures 2 and 10).

These complex mechanisms also result in scalpels which differ significantly from conventional scalpels in terms of their dimensions, weight and feel in the hand, and are therefore unfamiliar to the user. The mechanisms often result in bulky sheaths or protrusions, that impede the delicate movement of the instrument. Scalpels are used for highly intricate and delicate procedures, in which accuracy is extremely important. An unfamiliar instrument can be highly problematic for the user, impeding their performance and decreasing the accuracy and efficiency of the procedure. Use of the scalpel whilst avoiding contact with the release buttons can also prove awkward for the user. The result is that medical staff will often eschew a safety scalpel in favour of a more familiar, but less safe, conventional scalpel.

Accordingly it would be desirable to produce a scalpel that overcomes the problems outlined above.

According to the invention, there is provided a cutting instrument comprising: a handle having a distal end for supporting a blade; and a sheath movable longitudinally along the handle between a distal protective position to cover a blade at the distal end of the handle and a proximal use position to expose the blade for cutting; wherein the sheath and the handle have complementary engagement formations that define the protective position and the use position, the engagement formations comprising at least one engagement formation on the handle being a proximal recess and at least one engagement formation of the sheath being an inwardly-facing latch member cooperable with the proximal recess of the handle, the inwardly-facing latch member engaging with the proximal recess when the sheath is in the use position, and the proximal recess having proximal and distal walls oriented relative to a longitudinal axis of the handle such that longitudinal movement of the sheath is prevented proximally with respect to the use position and permitted distally with respect to the use position.

Preferably, the handle has a side-to-side thickness less than its depth in a direction transverse to its thickness. Also preferably, the engagement formation on the handle is located on at least one edge region of the handle.

In either of the aspects described above, the inwardly-facing latch member may be located on a cantilever on the sheath, which may be formed from a wall of the sheath. Preferably the sheath, the inwardly-facing latch member and the cantilever are formed as a single piece.

When the sheath is in the protective position the inwardly-facing latch member may engage with a distal recess.

The proximal recess may have a proximal wall that is substantially perpendicular relative to the longitudinal axis of the handle, and a distal wall at an acute angle relative to the longitudinal axis of the handle.

Optionally, the sheath may be further movable longitudinally along the handle to a locked position arranged distally with respect to the protective position. The inwardly-facing latch member may engage with a locking recess when the sheath is in the locked position.

Preferably, longitudinal movement of the sheath is prevented when the sheath is in the locked position. The locking recess may have proximal and distal walls oriented relative to a longitudinal axis of the handle such that longitudinal movement of the sheath is prevented when the sheath is in the locked position. For example, the locking recess may have proximal and distal walls that are substantially perpendicular relative to the longitudinal axis of the handle.

In the absence of a locking position, the distal recess may have proximal and distal walls oriented relative to a longitudinal axis of the handle such that longitudinal movement of the sheath is prevented distally with respect to the protective position and permitted proximally with respect to the protective position. For example, the distal recess may have a distal wall that is substantially perpendicular relative to the longitudinal axis of the handle, and a proximal wall that is at an acute angle relative to the longitudinal axis of the handle.

In the presence of a locking position, the distal recess may have proximal and distal walls oriented relative to a longitudinal axis of the handle such that longitudinal movement of the sheath is permitted distally and proximally with respect to the protective position. For example, the distal recess may have proximal and distal walls that are oriented at acute angles relative to the longitudinal axis of the handle.

Preferably the engagement formations on the handle are located on opposed edge regions of the handle. Also preferably, the sheath has at least one gripping formation on at least one external surface, which may be a wing arranged on a side of the sheath, and may be arranged at a proximal end of the sheath.

For ease of manufacture, the sheath and the gripping formation may be formed as a single piece. For example, the sheath may be formed from a sheet, and the gripping formation, the inwardly-facing latch member and the cantilever may be formed by bending the sheet, and the cantilever may be formed by cutting the sheet.

Optionally, the engagement formations may form resilient snap fittings.

In order that the invention may be more readily understood, reference will now be made, by way of example, to the accompanying drawings, in which:
Figure 1a is a perspective view of a scalpel in a first embodiment of the invention, with a sheath shown separately from the remainder of the scalpel;
Figure 1b is a perspective view of the scalpel of Figure 1 a with the sheath in a use position;
Figure 1c is a perspective view of the scalpel of Figure 1 a with the sheath in a protective position;
Figure 2a is a perspective view of a scalpel in a second embodiment of the invention, with a sheath shown separately from the remainder of the scalpel;
Figure 2b is a perspective view of the scalpel of Figure 2a with the sheath in a use position;
Figure 2c is a perspective view of the scalpel of Figure 2a with the sheath in a protective position; and
Figure 2d is a perspective view of the scalpel of Figure 2a with the sheath in a locked position.

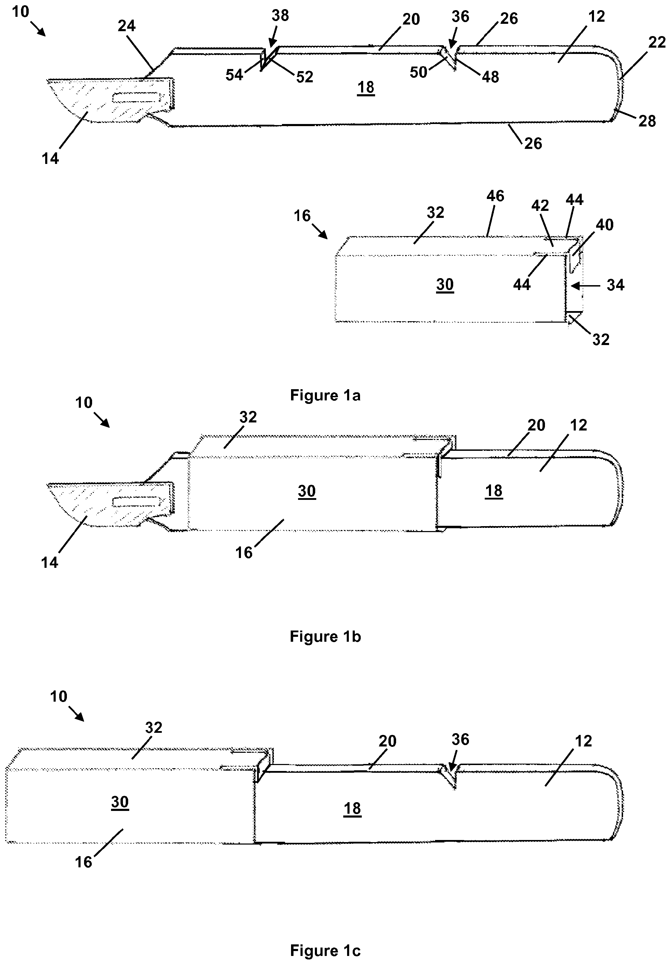

Referring to Fig. 1 of the drawings, a hand-held cutting instrument, exemplified here as a scalpel 10, has a handle 12, a blade 14, and a sheath 16. The handle 12 has two major sides 18, only one of which is shown, two minor sides 20, only one of which is shown, and two ends 22, 24. The major sides 18 are in generally parallel planes, oppositely disposed and broadly oblong in shape, with their long edges 26 defining the length of the handle 12 and their short edges 28 defining its depth. The perpendicular distance between the major sides 18 defines the side-to-side thickness of the handle 12. The minor sides 20 are also broadly oblong in shape and constitute top and bottom faces or edge regions of the handle 12. A proximal end 22 of the handle 12 is rounded and a distal end 24 of the handle 12 is tapered. The handle 12 is of similar size and shape to a conventional scalpel handle; it is of solid, continuous and essentially rectangular cross-section, and has a side-to-side thickness substantially less than its depth in a direction transverse to its thickness.

A blade 14 is at the distal end 24 of the handle 12. The blade 14 and handle 12 may be formed from a single piece, but in this example the blade 14 is conventionally formed from a separate piece that is permanently or removably attached to the handle 12.

Also provided is a sheath 16. The sheath 16 is a thin-walled, tubular, open-ended sleeve that has two major sides 30, of which only one is shown, and two minor sides 32, corresponding to the major sides 18 of the handle 12 and the minor sides 20 of the handle 12. The major sides 30 of the sheath 16 and the spacing between them define the length, depth and side-to-side width of the sheath 16 in the same way that the major sides 18 of the handle 12 define the length, depth and side-to-side thickness of the handle 12. The open ends 34 allow insertion of the handle 12 into the sheath 16.

Preferably, the depth and width of the sheath 16 are little greater than the depth and thickness of the handle 12, so that when the handle 12 is inserted into the sheath 16 the minor sides 20 and major sides 18 of the handle 12 lie closely against the interior of the respective minor sides 32 and major sides 30 of the sheath 16 as a sliding fit. In this way the presence of the sheath 16 does not significantly alter the dimensions, shape or weight distribution of the scalpel 10.

In this first embodiment of the invention, the sheath 16 is movable longitudinally along the handle 12 between first and second positions. The first position is a distal protective position where the sheath 16 covers the blade 14. The second position is a proximal use position where the blade 14 is exposed so that it can be used for cutting. The use and protective positions are defined by complementary engagement formations 36, 38 on the handle 12 and a complementary engagement formation 40 on the sheath 16. Advantageously, as shown, the engagement formations 36, 38 on the handle 12 are located on the top minor side 20 between the major sides 18.

In the embodiment shown in Fig. 1, the engagement formations 36, 38 on the handle 12 are recesses, exemplified as notches, located on the top minor surface 20. This means that the manufacturing process for the scalpel 10 is simple, and requires only a minor adaptation from the manufacturing process of a conventional scalpel. Alternatively, a conventional scalpel can be manufactured by a known process and adapted by cutting notches 36, 38 on the top minor side 20 at a later stage. The engagement formation 40 on the sheath 16 is an inwardly-facing latch member located on the corresponding minor side 32 of the sheath 16, which is cooperable with the notches 36, 38 of the handle 12. Preferably, as shown, the inwardly-facing latch member 40 is arranged on a cantilever 42. The cantilever 42 can be deflected inwardly and outwardly to effect respective inward and outward movement of the latch member 40, thereby facilitating its disengagement from the notches 36, 38.

Advantageously, and as shown, the cantilever 42 may be formed of one piece with the sheath 16. For example, the cantilever 42 shown in Fig. 1 a is formed from the wall of one of the minor sides 32 of the sheath 16. The proximal end of the wall is separated from the major sides 30 of the sheath 16 by cuts or slits 44 at the long edges 46 of the sheath 16. This provides a resilient wall portion that is capable of elastic deflection in the inward and outward directions, and can therefore act as the cantilever 42. The cantilever 42, being formed from the wall of the minor side 32, is biased to remain in substantially the same plane as the minor side 32. However, the cantilever 42 could instead be arranged so that it is biased to remain in a plane that is not the same as that of the minor side 32. The cantilever 42 need not be formed by the presence of cuts or slits 44; for example, the wall of the minor side 32 may instead extend longitudinally beyond the major sides 30 of the sheath 16 to provide the cantilever 42. The cantilever 42 does not need to be formed from a wall of a minor side 32, but could be formed from any wall of the sheath 16.

Advantageously, and as shown, the latch member 40 may also be formed of one piece with the cantilever 42. In the embodiment shown in Fig. 1a, the latch member 40 is a further continuation of the wall of the minor side 32 of the sheath 16, and extends perpendicularly from the cantilever 42. The latch member 40 could be formed, for example, by bending the end of the cantilever 42 so that it faces inwards, forming the latch member 40. In this embodiment the latch member 40 is perpendicular to the longitudinal axis of the sleeve 16, although it may be oriented at any suitable angle. Because the cantilever 42 is biased to remain in substantially the same plane as the wall of the minor side 32, the latch member 40, being perpendicular to the cantilever 42, is biased inwardly. The latch member 40 is therefore biased into engagement with the notches, 36, 38. In order to disengage the latch member 40 from the notches 36, 38 it is necessary to deflect the cantilever 42 outwardly.

In this way, formation of the cantilever 42 and the latch member 40 is simple; they can be formed, for example, by simple cutting and bending of the tubular sleeve 16. Alternatively the sleeve 16, cantilever 42 and latch member 40 could be cast or injection moulded from a simple mould. Advantageously, the shape and dimensions of the sleeve 16 remain substantially unaltered by the formation of the cantilever 42 and latch member 40.

The position of the notches 36, 38 on the minor side 32 of the handle 12 between the major sides 18 means that the notches 36, 38 can be deep enough to ensure that the inwardly-facing latch member 40 is stably engaged with the notches 36, 38, and that the force required to disengage it from the notches 36, 38 is sufficiently large that the sheath 16 will not be unintentionally moved from one position to another.

Figs. 1b and 1c show the scalpel 10 when the sheath 16 is in the use and protective positions respectively. When the sheath 16 is in the use position the latch member 40 is engaged with the first notch 36. When the sheath 16 is in the protective position the latch member 40 is engaged with the second notch 38. The sheath 16 is moved between the use and protective positions by the user applying a longitudinal force to push or pull the sheath 16 into the desired position with respect to the handle 12.

Preferably, as shown, the proximal walls 48, 52 and distal walls 50, 54 of the notches 36, 38 are oriented relative to the longitudinal axis of the handle 12 such that longitudinal movement of the sheath 16 is prevented proximally with respect to the use position and distally with respect to the protective position. In the embodiment shown in Fig. 1 the first notch 36 has a proximal wall 48 that is substantially perpendicular to the longitudinal axis of the handle 12, and a distal wall 50 that is at an acute angle to the longitudinal axis of the handle 12. The second notch has a distal wall 54 that is substantially perpendicular to the longitudinal axis of the handle 12, and a proximal wall 52 that is at an acute angle to the longitudinal axis of the handle 12.

The walls of the latch member 40 of the embodiment of Fig. 1 are perpendicular to the longitudinal axis of the handle 12 so that when the sheath 16 is pushed in a direction proximally with respect to the use position, the proximal wall 48 of the first notch 36 abuts the wall of the latch member 40, and there is no outward force acting on the latch member 40. Therefore the latch member 40 and cantilever 42 cannot deflect outwardly and longitudinal movement of the sheath 16 is prevented proximally with respect to the use position. By contrast, if the sheath 16 is pushed in a direction distally with respect to the use position, the acute angle of the distal wall 50 of the first notch 36 results in a outward component of the force acting on the latch member 40. When sufficient longitudinal force is applied, the latch member 40 and cantilever 42 are gradually deflected outward as the latch member 40 traverses the wall 50 and eventually the latch member 40 will disengage from the notch 48. Longitudinal movement of the sheath 16 is therefore allowed distally with respect to the use position.

Once the latch member 40 has traversed the distal wall 50, the latch member 40 and cantilever 42 are in a maximum outwardly deflected position. The sheath 16 may then be moved further distally with respect to the use position until the latch member 40 reaches the second notch 38. The sloping proximal wall 52 of the second notch 38 allows gradual inward movement of the latch member 40 by reducing the deflection of the cantilever 42. When the latch member 40 is fully engaged with the notch 38 the latch member 40 and cantilever 42 are in a minimum deflected position. In the embodiment shown in Fig. 1 there is no deflection of the cantilever 42 when the latch member 40 is engaged with the notch 38. Longitudinal movement of the sheath 16 is therefore permitted proximally with respect to the protective position because of the sloping proximal wall 52 of the second notch 38, and is prevented distally with respect to the protective position by the perpendicular distal wall 54 of the second notch 38.

Thus, the inwardly-facing latch member 40 cannot move proximally with respect to the first notch 36, or distally with respect to the second notch 38, but can move between the two notches 36, 38. The sheath 16 can therefore move between the two positions, but cannot be removed from the handle 12 without applying a substantial force, or otherwise releasing the latch member 40 from a notch 36, 38.

In combination, the engagement formations 36, 38 on the handle 12 and the engagement formation 40 on the sheath 16 form resilient snap fittings, so that when the latch member 40 engages with a notch 36, 38 an audible click is produced, indicating to the user by sound and by feel that the sheath 16 is in its chosen position.

In use, the scalpel 10 as shown in Fig. 1 is presented with the sheath 16 initially in the protective position. The scalpel 10 may be passed amongst medical staff without risk of injury from the blade 14. The user moves the sheath 16 to the use position and uses the scalpel 10 for cutting. The compact construction of the sheath 16 and the simple mechanism of the engagement formations 36, 38, 40 means that when the sheath 16 is in the use position the instrument 10 does not differ significantly from a conventional scalpel in terms of its dimensions, shape, weight or feel in the hand. There are no release buttons that may cause accidental sheathing or unsheathing of the blade 14, and the user's hand and fingers do not come into contact with any unfamiliar features. After use, the sheath 16 can be returned to the protective position, and the scalpel 10 can be passed safely amongst medical staff once more. At the end of the procedure the scalpel 10 can be discarded, or it can be sterilised and re-used. Typically scalpels are disposable and so the simplicity of the present invention is highly advantageous.

Fig. 2 shows an alternative and preferred embodiment of the invention. In this embodiment the sheath 16 is further movable longitudinally along the handle 12 to a third, locked position. Figs. 2b and 2c show the use and protective positions respectively. Fig. 2d shows the scalpel 10 with the sheath 16 in the locked position. When the sheath 16 is in the locked position the inwardly-facing latch member 40 is engaged with a third notch 56.

The third notch 56 has a distal wall 58 and a proximal wall 60, both oriented with respect to the longitudinal axis of the handle 12 such that longitudinal movement of the sheath 16 in either direction is prevented when the sheath 16 is in the locked position. In the example shown in Fig. 2 both walls 58, 60 are substantially perpendicular to the longitudinal axis of the handle 12, so that when the user pushes or pulls the sheath 16 the inwardly-facing latch member 40 abuts the walls 58, 60 and cannot disengage from the notch 56.

In this embodiment the first notch 36 has a proximal wall 48 substantially perpendicular to the longitudinal axis of the handle 12, and a distal wall 50 at an acute angle to the longitudinal axis of the handle 12 so that the sheath 16 cannot be moved proximally with respect to the use position. Both the proximal and distal walls 52, 54 of the second notch are inclined at an acute angle to the longitudinal axis of the handle 12 to allow the sheath 16 to move between the use position and the protective position, and from the protective position to the locked position.

In use, the scalpel 10 as shown in Fig. 2 is presented with the sheath 16 initially in the protective position so that the scalpel 10 may be passed amongst medical staff without risk of injury from the blade 14. The user then moves the sheath 16 to the use position and uses the scalpel 10 for cutting. After use the sheath 16 can be returned to the protective position, and the scalpel 10 can be passed amongst medical staff once more. When the scalpel 10 is no longer required, for example at the end of the procedure, the sheath 16 can be moved to the locked position, where the sheath 16 is no longer movable longitudinally, for safe disposal.

In any embodiment of the invention the engagement formations 36, 38, 56 on the handle 12 may be replicated on opposite minor sides 20 of the handle 12, and the engagement formations on the sheath 40 may also be replicated on corresponding opposite minor sides 32 of the sheath 16, as shown in Fig. 2. This increases the stability of the sheath 16 when in one of the specified positions. One or more gripping formations 62 may also be provided on the sheath 16, as also shown in Fig. 2, so that it is easier for the user to apply the longitudinal force necessary to push or pull the sheath 16 between positions.

The gripping formations 62 shown in Fig. 2 are wings positioned at the proximal end of the sheath 16, on its major sides 30. In this way the gripping formations 62 are unlikely to encounter the user's hands or fingers during use of the scalpel 10 for cutting because the user's fingers will typically grip a distal portion of the handle 12 during use. The gripping formations 62 may be made from a single piece with the sheath 16. For example, the walls of the major sides 30 of the sheath 16 may be extended beyond the minor sides 32 of the sheath 16, and bent outwardly to form wings.

In the example shown in Fig. 2 the sheath 16, the inwardly-facing latch members 40, the cantilevers 42 and the gripping formations 62 are all formed from a single piece.

The piece may be made from metal, plastic or any other suitable material. Preferably, the piece is formed from a sheet such as sheet metal by cutting and/or bending the sheet to form the desired features. Alternatively, the sheath 16 may be made from metal by alternative methods such as casting and machining. The sheath 16 may also be made from a non-metallic material such as a plastic by methods such as injection moulding.

It will be appreciated that many variations in the design of the instrument are possible within the scope of the disclosure. For example, the engagement formations 36, 38, 56 on the handle 12 may be outwardly-facing latch members and the engagement formations 40 on the sheath 16 may be complementary recesses. Alternatively the engagement formations 36, 38, 56, 40 may be latch members on both the sheath 16 and the handle 12.

The instrument need not be a scalpel intended for surgical use, but may be a cutting instrument for other purposes such as a hand-held knife for cutting cardboard, paper and light wood, or for food preparation.

## Claims

1. A cutting instrument (10) comprising:
a handle (12) having a distal end for supporting a blade; and
a sheath (16) movable longitudinally along the handle (12) between a distal protective position to cover a blade (14) at the distal end of the handle (12) and a proximal use position to expose the blade (14) for cutting;
wherein the sheath (16) and the handle (12) have complementary engagement formations (36, 38, 40) that define the protective position and the use position, the engagement formations (36, 38, 40) comprising at least one engagement formation on the handle (12) being a proximal recess (36) and at least one engagement formation (40) of the sheath (16) being an inwardly-facing latch member (40) cooperable with the proximal recess (36) of the handle (12), the inwardly-facing latch member (40) engaging with the proximal recess (36) when the sheath (16) is in the use position, and the proximal recess (36) having proximal and distal walls (48, 50) oriented relative to a longitudinal axis of the handle (12) such that longitudinal movement of the sheath (16) is prevented proximally with respect to the use position and permitted distally with respect to the use position.

2. The cutting instrument (10) of claim 1 wherein the handle (12) has a side-to-side thickness less than its depth in a direction transverse to its thickness.

3. The cutting instrument (10) of claim 1 or claim 2 wherein at least one of the engagement formations (36, 38) on the handle (12) is located on at least one edge region (20) of the handle (12).

4. The cutting instrument (10) of any of claims 1 to 3 wherein the inwardly-facing latch member (40) is located on a cantilever (42) on the sheath (16).

5. The cutting instrument (10) of claim 4 wherein the cantilever (42) is formed from a wall of the sheath (16).

6. The cutting instrument (10) of claim 4 or claim 5 wherein the sheath (16), the inwardly-facing latch member (40) and the cantilever (42) are formed as a single piece.

7. The cutting instrument (10) of any of claims 1 to 6 wherein the inwardly-facing latch member (40) engages with a distal recess (38) when the sheath (16) is in the protective position.

8. The cutting instrument (10) of any of claims 1 to 7 wherein the proximal recess (36) has a proximal wall (48) that is substantially perpendicular relative to the longitudinal axis of the handle (12), and a distal wall (50) at an acute angle relative to the longitudinal axis of the handle (12).

9. The cutting instrument (10) of any of claims 1 to 8 wherein the sheath (16) is further movable longitudinally along the handle (12) to a locked position arranged distally with respect to the protective position.

10. The cutting instrument (10) of claim 9, wherein the inwardly-facing latch member (40) engages with a locking recess (56) when the sheath (16) is in the locked position.

11. The cutting instrument (10) of claim 10, wherein the locking recess (56) has proximal and distal walls (58, 60) oriented relative to a longitudinal axis of the handle (12) such that longitudinal movement of the sheath (16) is prevented when the sheath (16) is in the locked position.

12. The cutting instrument (10) of claim 7 or claim 8 wherein the distal recess (38) has proximal and distal walls (52, 54) oriented relative to a longitudinal axis of the handle (12) such that longitudinal movement of the sheath (16) is prevented distally with respect to the protective position and permitted proximally with respect to the protective position.

13. The cutting instrument (10) of claim 12 wherein the distal recess (38) has a distal wall (54) that is substantially perpendicular relative to the longitudinal axis of the handle (12), and a proximal wall (52) that is at an acute angle relative to the longitudinal axis of the handle (12).

14. The cutting instrument (10) of any of claims 9 to 11 wherein the distal recess (38) has proximal and distal walls (52, 54) oriented relative to a longitudinal axis of the handle (12) uch that longitudinal movement of the sheath (16) is permitted distally and proximally with respect to the protective position.

15. The cutting instrument (10) of any preceding claim wherein the engagement formations (36, 38, 56) on the handle (12) are located on opposed edge regions (20) of the handle (12).

## Patentansprüche

1. Schneidvorrichtung (10), Folgendes umfassend:
einen Griff (12) mit einem distalen Ende zum Lagern einer Klinge; und
eine Scheide (16), die der Länge nach entlang des Griffs (12) zwischen einer distalen Schutzstellung zum Abdecken einer Klinge (14) am distalen Ende des Griffs (12) und einer proximalen Verwendungsstellung zum Freilegen der Klinge (14) zum Schneiden beweglich ist;
wobei die Scheide (16) und der Griff (12) komplementäre Eingriffsausbildungen (36, 38, 40), die die Schutzstellung und die Verwendungsstellung definieren, aufweisen, wobei die Eingriffsausbildungen (36, 38, 40) Folgendes umfassen: wenigstens eine Eingriffsausbildungen auf dem Griff (12), die eine proximale Vertiefung (36) ist, und wenigstens eine Eingriffsausbildung (40) der Scheide (16) ein nach innen weisendes Einrastelement (40) ist, das mit der proximalen Vertiefung (36) des Griffs (12) zusammenwirken kann, wobei das nach innen weisende Einrastelement (40) in die proximale Vertiefung (36) eingreift, wenn die Scheide (16) sich in der Verwendungsstellung befindet, und die proximale Vertiefung (36) proximale und distale Wände (48, 50) aufweist, die bezogen auf eine Längsachse des Griffs (12) derart ausgerichtet sind, dass eine Längsbewegung der Scheide (16) proximal bezogen auf die Verwendungsstellung verhindert wird und distal bezogen auf die Verwendungsstellung ermöglicht wird.

2. Schneidvorrichtung (10) nach Anspruch 1, wobei der Griff (12) eine Dicke von Seite zu Seite aufweist, die geringer ist als ihre Tiefe in einer Richtung quer zu ihrer Dicke.

3. Schneidvorrichtung (10) nach Anspruch 1 oder 2, wobei wenigstens eine der Eingriffsausbildungen (36, 38) auf dem Griff (12) sich auf wenigstens einem Kantenbereich (20) des Griffs (12) befindet.

4. Schneidvorrichtung (10) nach einem der Ansprüche 1 bis 3, wobei das nach innen weisende Einrastelement (40) sich auf einem Ausleger (42) auf der Scheide (16) befindet.

5. Schneidvorrichtung (10) nach Anspruch 4, wobei der Ausleger (42) von einer Wand der Scheide (16) aus gebildet ist.

6. Schneidvorrichtung (10) nach Anspruch 4 oder 5, wobei die Scheide (16), das nach innen weisende Einrastelement (40) und der Ausleger (42) als ein einziges Stück ausgebildet sind.

7. Schneidvorrichtung (10) nach einem der Ansprüche 1 bis 6, wobei das nach innen weisende Einrastelement (40) in eine distale Vertiefung (38) eingreift, wenn die Scheide (16) sich in der Schutzstellung befindet.

8. Schneidvorrichtung (10) nach einem der Ansprüche 1 bis 7, wobei die proximale Vertiefung (36) eine proximale Wand (48), die im Wesentlichen senkrecht bezogen auf die Längsachse des Griffs (12) ist, und eine distale Wand (50) in einem spitzen Winkel bezogen auf die Längsachse des Griffs (12) aufweist.

9. Schneidvorrichtung (10) nach einem der Ansprüche 1 bis 8, wobei die Scheide (16) ferner der Länge nach entlang des Griffs (12) in eine verriegelte Stellung, die distal bezogen auf die Schutzstellung angeordnet ist, beweglich ist.

10. Schneidvorrichtung (10) nach Anspruch 9, wobei das nach innen weisende Einrastelement (40) in eine Verriegelungsvertiefung (56) eingreift, wenn die Scheide (16) sich in der Schutzstellung befindet.

11. Schneidvorrichtung (10) nach Anspruch 10, wobei die Verriegelungsvertiefung (56) eine proximale und eine distale Wand (58, 60) aufweist, die bezogen auf eine Längsachse des Griffs (12) derart ausgerichtet sind, dass eine Längsbewegung der Scheide (16) verhindert wird, wenn sich die Scheide (16) in der verriegelten Stellung befindet.

12. Schneidvorrichtung (10) nach Anspruch 7 oder 8, wobei die distale Vertiefung (38) eine proximale und eine distale Wand (52, 54) aufweist, die bezogen auf eine Längsachse des Griffs (12) derart ausgerichtet sind, dass eine Längsbewegung der Scheide (16) distal bezogen auf die Schutzstellung verhindert wird und proximal bezogen auf die Schutzstellung ermöglicht wird.

13. Schneidvorrichtung (10) nach Anspruch 12, wobei die distale Vertiefung (38) eine distale Wand (54), die im Wesentlichen senkrecht bezogen auf die Längsachse des Griffs (12) ist, und eine proximale Wand (52), die in einem spitzen Winkel bezogen auf die Längsachse des Griffs (12) steht, aufweist.

14. Schneidvorrichtung (10) nach einem der Ansprüche 9 bis 11, wobei die distale Vertiefung (38) eine proximale und eine distale Wand (52, 54) aufweist, die bezogen auf eine Längsachse des Griffs (12) derart ausgerichtet sind, dass eine Längsbewegung der Scheide (16) distal und proximal bezogen auf die Schutzstellung ermöglicht wird.

15. Schneidvorrichtung (10) nach einem der vorhergehenden Ansprüche, wobei die Eingriffsausbildungen (36, 38, 56) auf dem Griff (12) sich auf einander gegenüberliegenden Kantenbereichen (20) des Griffs (12) befinden.

## Revendications

1. Instrument de découpe (10) comprenant :
une poignée (12) ayant une extrémité distale pour supporter une lame ; et
une gaine (16) pouvant se déplacer longitudinalement le long de la poignée (12) entre une position distale de protection pour recouvrir une lame (14) à l extrémité distale de la poignée (12) et une position proximale d utilisation afin d exposer la lame (14) pour la découpe ;
dans lequel la gaine (16) et la poignée (12) ont des formations de prise complémentaires (36, 38, 40) qui définissent la position de protection et la position d utilisation, les formations de prise (36, 38, 40) comprenant au moins une formation de prise sur la poignée (12) qui est un évidement proximal (36) et au moins une formation de prise (40) de la gaine (16) qui est un élément de verrouillage orienté vers l intérieur (40) pouvant coopérer avec l évidement proximal (36) de la poignée (12), l élément de verrouillage orienté vers l intérieur (40) venant en prise avec l évidement proximal (36) lorsque la gaine (16) est dans la position d utilisation, et l évidement proximal (36) présente des parois proximale et distale (48, 50) orientées par rapport à un axe longitudinal de la poignée (12) de sorte que le mouvement longitudinal de la gaine (16) soit empêché de manière proximale par rapport à la position d utilisation et permis de manière distale par rapport à la position d utilisation.

2. Instrument de découpe (10) selon la revendication 1 dans lequel la poignée (12) a une épaisseur latérale inférieure à sa profondeur dans une direction transversale à son épaisseur.

3. Instrument de découpe (10) selon la revendication 1 ou la revendication 2 dans lequel au moins une des formations de prise (36, 38) sur la poignée (12) est placée sur au moins une zone de bord (20) de la poignée (12).

4. Instrument de découpe (10) selon l une quelconque des revendications 1 à 3 dans lequel l élément de verrouillage orienté vers l intérieur (40) est placé sur une extension (42) sur la gaine (16).

5. Instrument de découpe (10) selon la revendication 4 dans lequel l extension (42) est formée à partir d une paroi de la gaine (16).

6. Instrument de découpe (10) selon la revendication 4 ou la revendication 5 dans lequel la gaine (16), l élément de verrouillage orienté vers l intérieur (40) et l extension (42) sont formés d une seule pièce.

7. Instrument de découpe (10) selon l une quelconque des revendications 1 à 6 dans lequel l élément de verrouillage orienté vers l intérieur (40) vient en prise avec un évidement distal (38) lorsque la gaine (16) est dans la position de protection.

8. Instrument de découpe (10) selon l une quelconque des revendications 1 à 7 dans lequel l évidement proximal (36) a une paroi proximale (48) qui est sensiblement perpendiculaire à l axe longitudinal de la poignée (12), et une paroi distale (50) orientée selon un angle aigu par rapport à l axe longitudinal de la poignée (12).

9. Instrument de découpe (10) selon l une quelconque des revendications 1 à 8 dans lequel la gaine (16) peut en outre se déplacer longitudinalement le long de la poignée (12) jusqu à une position verrouillée agencée de manière distale par rapport à la position de protection.

10. Instrument de découpe (10) selon la revendication 9, dans lequel l élément de verrouillage orienté vers l intérieur (40) vient en prise avec un évidement de verrouillage (56) lorsque la gaine (16) est dans la position verrouillée.

11. Instrument de découpe (10) selon la revendication 10, dans lequel l évidement de verrouillage (56) a des parois proximale et distale (58, 60) orientées par rapport à un axe longitudinal de la poignée (12) de sorte que le mouvement longitudinal de la gaine (16) soit empêché lorsque la gaine (16) est dans la position verrouillée.

12. Instrument de découpe (10) selon la revendication 7 ou la revendication 8 dans lequel l évidement distal (38) a des parois proximale et distale (52, 54) orientées par rapport à un axe longitudinal de la poignée (12) de sorte que le mouvement longitudinal de la gaine (16) soit empêché de manière distale par rapport à la position de protection et permis de manière proximale par rapport à la position de protection.

13. Instrument de découpe (10) selon la revendication 12 dans lequel l évidement distal (38) a une paroi distale (54) qui est sensiblement perpendiculaire à l axe longitudinal de la poignée (12), et une paroi proximale (52) orientée selon un angle aigu par rapport à l axe longitudinal de la poignée (12).

14. Instrument de découpe (10) selon l une quelconque des revendications 9 à 11 dans lequel l évidement distal (38) a des parois proximale et distale (52, 54) orientées par rapport à un axe longitudinal de la poignée (12) de sorte que le mouvement longitudinal de la gaine (16) soit permis de manière distale et de manière proximale par rapport à la position de protection.

15. Instrument de découpe (10) selon l une quelconque des revendications précédentes dans lequel les formations de prise (36, 38, 56) sur la poignée (12) sont placées sur des zones de bord (20) opposées de la poignée (12).
